# EUROPEAN PATENT SPECIFICATION

(11) **EP 1 948 207 B1**
(45) Date of publication and mention of the grant of the patent: **18.11.2015**
(21) Application number: 05818384.9
(22) Date of filing: 20.12.2005
(51) Int. Cl.: A61P 37/08, A61K 36/185, A61K 36/00

(54) **FAGONIA BRUGUIERI FREEZE-DRIED EXTRACT AS ANTI-ALLERGIC TREATMENT**
GEFRIERGETROCKNETER EXTRAKT AUS FAGONIA BRUGUIERI ALS ANTIALLERGISCHE BEHANDLUNG
EXTRAIT LYOPHILISÉ DE FAGONIA BRUGUIERI COMME TRAITEMENT ANTI-ALLERGIQUE

(43) Date of publication of application: 30.07.2008
(73) Proprietor: King Saud University, 11421 Riyadh (SA)
(72) Inventor: Al-Yahya, Mohammed Abdulaziz, Riyadh 11451 (SA); EITahir, Kamel Eldin Hussein, Khartoum North Safia (SD)
(74) Representative: Scholz, Volker
(86) International application number: PCT/IB2005/003712
(87) International publication number: WO 2007/072100

(56) References cited:
- EP-A- 0 265 662
- WO-A-87/06833
- GEDARA SAHAR R ET AL: "New erythroxane-type diterpenoids from Fagonia boveana (Hadidi) Hadidi & Graf." ZEITSCHRIFT FÜR NATURFORSCHUNG. C, JOURNAL OF BIOSCIENCES. 2003 JAN-FEB, vol. 58, no. 1-2, January 2003 (2003-01), pages 23-32, XP009069990 ISSN: 0341-0382
- GEHLOT D ET AL: "Antibacterial effect of some leaf extracts on Salmonella typhi." INDIAN JOURNAL OF MEDICAL SCIENCES. MAR 2000, vol. 54, no. 3, March 2000 (2000-03), pages 102-105, XP009069991 ISSN: 0019-5359
- ABDEL-KADER M S ET AL: "Erythroxan diterpenes from Fagonia glutinosa." PLANTA MEDICA. AUG 1997, vol. 63, no. 4, August 1997 (1997-08), pages 374-376, XP009069993 ISSN: 0032-0943
- ANSARI A A ET AL: "ISOLATION AND CHARACTERIZATION OF TWO SAPONINS FROM FAGONIA-INDICA" PHYTOCHEMISTRY (OXFORD), vol. 26, no. 5, 1987, pages 1487-1490, XP009070001 ISSN: 0031-9422
- ASIF SAEED M ET AL: "Effects of Fagonia cretica L. constituents on various haematological parameters in rabbits." JOURNAL OF ETHNOPHARMACOLOGY. APR 2003, vol. 85, no. 2-3, April 2003 (2003-04), pages 195-200, XP009069999 ISSN: 0378-8741
- ABDEL-KHALIK S M ET AL: "Further saponins from Fagonia cretica." DIE PHARMAZIE. MAR 2001, vol. 56, no. 3, March 2001 (2001-03), pages 247-250, XP001247413 ISSN: 0031-7144

## Description

### Summary

The whole plant *Fogonia **bruguieri*** DC., family Zygophylleceae was extracted with boiling water and freeze-dried. The LD₅₀ values of the dried extract were 11.5 and 10.75 g/kg i.p. in mice and rats, respectively. Treatment of albino guinea-pigs with the extract in doses of 200 mg/kg (i-v.) or orally antagonized histamine (20 µg/kg i.v.) - and capsaicin (100 µg/kg i.v.) - induced bronchoconstriction without affecting that induced with ACh and 5-HT. The percentage antagonisms were 72 ± 0.9 and 65 ± 4% against histamine and capsaicin, respectively (P < 0.01, N = 10). Exposure of conscious guinea-pigs to histamine aqueous aerosols (10 mg/ml) induced initial graspings and reversible loss of consciousness within 5 minutes. Treatment of the guinea-pigs with the extract in doses of 1.25 g/kg (i.p.) for 20 minutes or orally for 2 hours protected significantly the animals against histamine-induced grasps and loss of consciousness (P < 0.01, N = 11). *Fagonia bruguieri* freeze-dried aqueous extract is claimed to be a component of a pharmaceutical product for the treatment of histamine- and capsaicin-induced bronchoconstriction.

### 1. Introduction

The genus *Fagonia* includes about 35 species that are distributed in the deserts and dry areas in India, tropical Africa, Chile and South West USA. Many of the species are spiny herbs or sub shrubs (Chopra *et al.,* 1982). Some of *Fagonia* species were investigated chemically and these investigations revealed the presence of flavonol glycosides in *F. glutinosa, F. isothricha* (Al-Wakeel and Shahnaz, 1992); *F*. *thebica* (Al-Wakeel *et al.,* 1988), *F. mollis* (AI-Wakeel *et al.,* 1987), *F. indica* (El-Hadidi *et al*., 1988) and *F. arabica* (ElNegoumy *et al.,* 1986). Furthermore, two saponins were isolated from *F. indica* (Ansari *et al.,* 1987), docosyl docusanoate was isolated from *F. cretica* (Hamid *et al*., 1989), the sapogenins-fagoenin and betulin from *F. cretica* (Ali and Hameed, 1967), the triterpenoid saponins-indica saponin A & B from *F. indica* (Shaker *et al.,* 1999), nahagenin, hederagenin, ursolic acid and pinitol from *F. indica* (Atta-ur-Rahman *et al*., 1982; 1984) and 27-hydroxyoleanolic acid and 27-ursolic acid from *F. arabica* (Miyase *et al*., 1996). The flavonoids showed anti-microbial activity (Hash and Nag, 1988) and *F*. *cretica* saponins were shown to suppress serum prolactin and thyroid-stimulating hormone and to increase cortisol levels in rabbits (Saeed, 1999).

One of the Fagoni species found in Saudi Arabia is *F. bruguieri.* Chemical analysis of the aerial parts of the plant revealed the presence of erythroxan-type diterpenes such as 7β-hydroxyfagonene (15,16-dihydroxy) 7β-hydroxy-cis-enterythox-3-ene (Abdel-Kader *et al*., 1994), fagonene (15, 16-dihydroxy-7-oxo-cis-enterythrox-3-ene and 16-O-acetylfagonone (Abdel-Kader *et al.,* 1993). Literature survey revealed the absence of pharmacological investigations of this plant. Pilot pharmacological investigations revealed the antihistaminic activity of its aqueous extract. It was thus thought of interest to investigate this property in guinea-pigs together with its acute toxicity in mice and rats.

### 2. Methods

### 2.1 Extraction of the Plant

The whole plant *F. bruguieri* was collected from the central areas of Saudi Arabia in March 2004 and the plant was identified by the College of Pharmacy, King Saud University, taxonomist. A voucher sample is preserved at the College herbarium in Riyadh, Saudi Arabia. The plant was dried at room temperature and extracted with boiling water. The aqueous extract was then filtered and freeze-dried. The freeze-dried powder was then used to prepare the different aqueous concentrations used in this study.

### 2.2 Phytochemical Screening of the Freeze-dried Extract

Chemical investigations of the various plant chemicals were performed as described by Wagner (1983).

### 2.3 Determination of the Lethal Dose 50 (LD₅₀)

Male albino Swiss mice (25 g body weight) and male Wistar rats rats (250 g body weight) were divided into 6 groups each (N = 10 animals per group). The groups were then administered different doses of the re-constituted aqueous freeze-dried extract in doses that ranged from (0.5-16 g/kg intraperitoneally). All of the animals were observed for abnormal behaviours and death for 72 hours. The percentage deaths in each group were recorded and converted into probits to calculate the LD₅₀ values as reported by Ghosh (1984).

### 2.4 Preparation of Guinea-pigs for Measurement of the Intra-tracheal Pressure

Male albino guinea-pigs (800 g body weight) were anaesthetized with aqueous urethane (25% w/v) in a dose of (1.5 g/kg, i.p.) and prepared for measurement of the intra-tracheal pressure by a modification of the method described by ELTahir *et al.* (1993). In brief, following anaesthesia, each animal was injected i.p. with the non-selective β-adrenoceptor blocker propranolol in a dose of 1 mg/kg and methylene blue - the NO antagonist - in a dose of 20 mg/kg i.p. Thereafter, the external jugular vein and the trachea were cannulated. For cannulation of the trachea a T-shaped cannula was used. One limb of the cannula was connected to an ITT pressure transducer attached to a Narco-Physiograph Recorder (MK-1-V-P-Narco-Biosyste ms, USA) and the other limb was connected *via* plastic tubings to an air pump rodent ventilation (Scientific and Research Instruments, Ltd, UK) for artificial respiration using room air and a tidal volume of 6 ml air per kg as reported by Tocker *et al.* (1990). The tracheal pressure was then displayed onto the recording system. The effect of each dose on the intra-tracheal pressure in mm Hg was quantified by the aid of the pressure calibration system built-in the Physiograph coupler. Initially, dose response curves for the bronchoconstricting effect of histamine, ACh, 5-HT and capsaicin using doses in the range (10-100 µg/kg i.v.). The submaximal dose of each agent was then selected. Each bronchoconstrictor was studied in a different animal (N = 10).

To investigate the influence of the extract on the bronchoconstrictors, the submaximal dose of the bronchoconstrictor was first injected and its effect recorded. The animal was allowed to recover. Thereafter, the selected dose of the extract (200 mg/kg) was injected i.v. and 5 minutes later, the submaximal dose of the bronchoconstrictor was injected. The magnitudes of the induced increases in the intra-tracheal pressures were recorded and calculated. The influence of the extract on the induced bronchoconstriction was then calculated as percentage antagonism. To test the effect of the oral treatment, the extract (100 or 200 mg/kg) was administered orally by gavage before anaesthesia and 2 hours later the effect of the bronchoconstrictor was studied.

### 2.5 Exposure of Guinea-pigs to Histamine Aerosol

Male albino guinea-pigs (800 g body weight) were used in this experiment. Each animal was placed in a 25-litre descicator-like glass container provided with inlets. One inlet was used to introduce air and another was used to introduce the histamine aerosol (10 mg/ml in water) using a modified glass nebulizer. To start the experiment the animal was placed into the closed aerated glass container and then histamine spraying was started at a constant pressure. The spraying was continued until the animal lost consciousness as indicated by its falling down. The animal was continuously observed and the onset time of grasping (difficulty in respiration) and all subsequent grasps were recorded in minutes following exposure to histamine spray. Then the time of complete loss of consciousness and falling of the animal was recorded. A group of animals (N = 11) was injected with the extract in a dose of 1.25 g per kg i.p. and twenty minutes later was exposed to histamine spray. Times for the different grasps and total loss of consciousness were then recorded and compared with those observed in the control untreated animals (N = 11). The same procedure was repeated after the oral treatment of a group of animals by gavage using a dose of 200 mg/kg. The animals were exposed to histamine two hours after treatment with extract.

### 2.6 Materials

ACh, histamine, 5-HT, capsaicin, propranolol, methylene blue, heparin were all obtained from Sigma Chemical Company (St. Louis, MO, USA). Capsaicin was dissolved in ethyl alcohol 10 mg/ml and diluted with distilled water. All other chemicals were dissolved in distilled water.

### 2.7 Statistical Analysis

All values were reported as mean ± s.c. mean with N = number of animals tested per group. Significant differences between the different treatments were analyzed using Student's 't'-test or ANOVA as appropriate.

### 3. Results

### 3.1 Phytochemical Analysis

Phytochemical analysis of the freeze-dried aqueous extract revealed the presence of alkaloids, flavonoids and tannins. Other chemicals such as anthracene derivatives, cardiac glycosides, saponins, sterols, or triterpenes and coumarins were not detected.

### 3.2 LD₅₀ Values

The extract was greatly tolerated by both mice and rats. This was reflected by the high LD₅₀ values which were 11.5 and 10.75 g/kg i.p. in mice and rats, respectively.

### 3.3 Effect of the extract on the Bronchoconstrictors-induced Increase in the Intra-tracheal Pressure

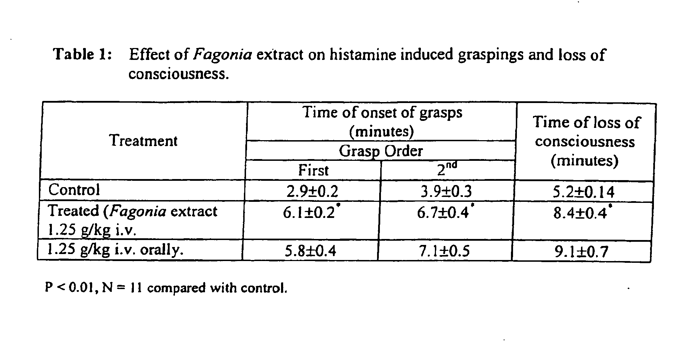

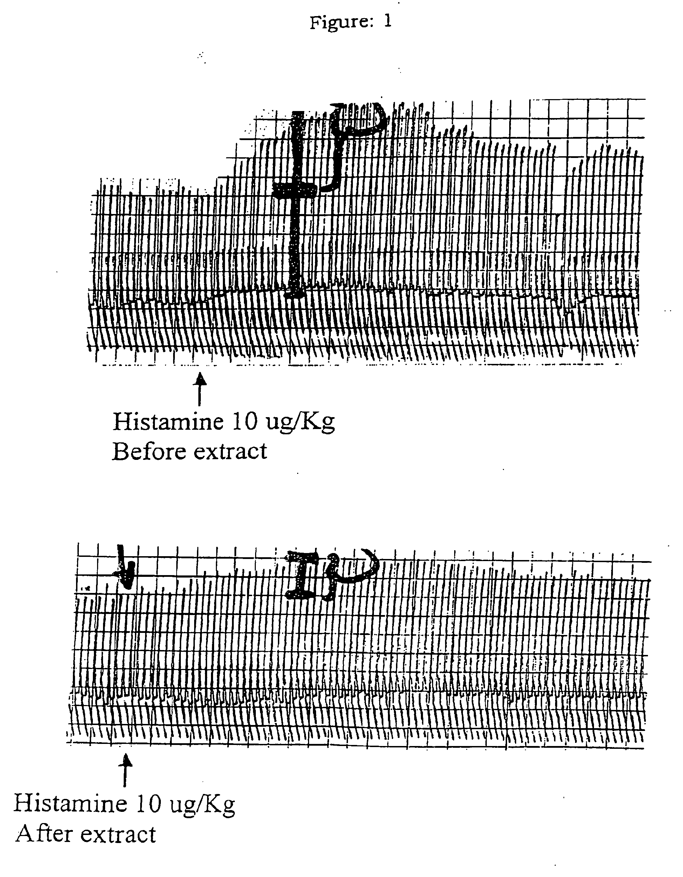

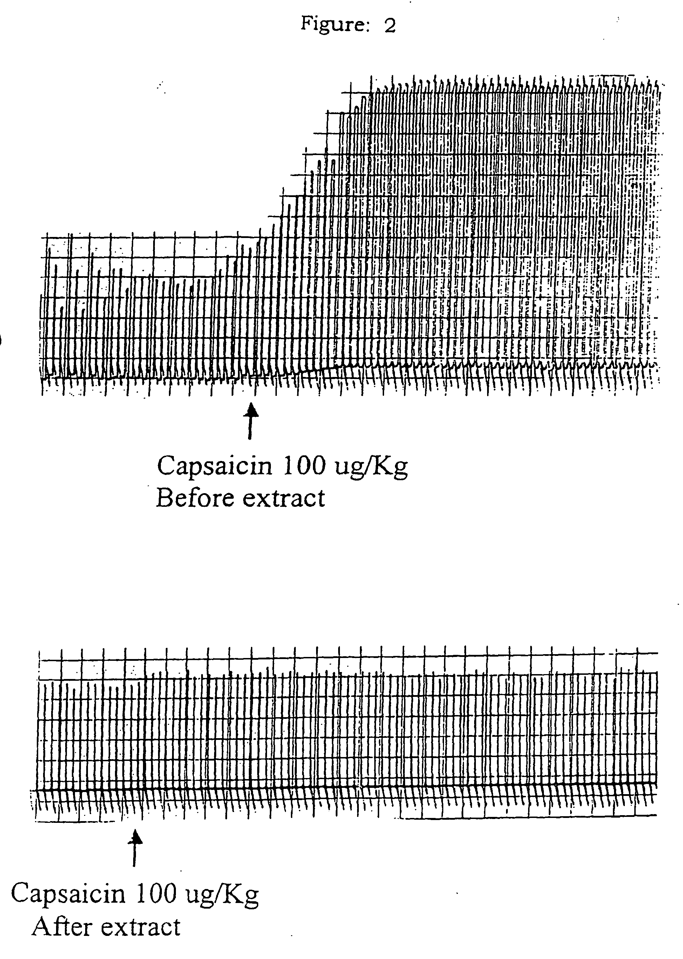

Administration of ACh, 5-HT, histamine and capsaicin intravenously into the guinea-pigs in the dose range 5-100 µg/kg induced dose-dependent bronchoconstrictions. Intravenous or oral treatment of the animals with the extract in the dose range of 50-100 mg/kg for 5 minutes following the i.v. treatment produced non-significant antagonisms to the bronchoconstriction induced by the submaximal doses of the four bronchoconstrictors. However, treatment of the animals with the extract in doses of (200 mg/kg i.v.) for 5 minutes or orally for 2 hours significantly suppressed histamine (20 µg/kg i.v.)- and capsaicin (100 µg/kg i.v.)-induced bronchoconstrictions. The mean percentage antagonism to histamine was 72 ± 0.9 and to capsaicin was 65 ± 4% (P < 0.01, N = 10). Figures 1 and 2 depict the effects of the (i.v.) extract against histamine and capsaicin, respectively. The extract did not antagonize ACh and 5-HT.

### 3.4 The effect of the Extract on Histamine-induced Loss of Consciousness and Graspings in Guinea-pigs.

Exposure of conscious guinea-pigs to histamine (aqueous aerosols) (10 mg/ml) induced initial graspings (bronchoconstriction) followed by reversible loss of consciousness. Treatment of the animals with the extract orally or i.v. in doses up to 0.5 g/kg did not induce significant antagonism to histamine-induced grasps or loss of consciousness. However, when the animals were treated with the extract in doses of 1.25 g/kg i.p. for 20 minutes or orally 2 hours before exposure to the histamine aerosol there were significant prolongations of the times for onsets of the grasps and the loss of consciousness (P < 0.01, N = 11). The cumulative results are shown in Table 1.

### 4. Discussion

The present study demonstrated the ability of the freeze-dried extract of *Fagonia bruguieri* to antagonize histamine and capsaicin induced bronchoconstriction. It did not antagonize ACh or 5-HT induced bronchoconstriction. The results also revealed the high safety of the extract as reflected by its high therapeutic index (> 8). Confirmation to histamine antagonism was also revealed by the protective effect of the extract on histamine antagonism was also revealed by the protective effect of the extract on histamine-induced grasps and loss of consciousness in guinea-pigs. Such inherent ability may be related to the flavonoids, tannins or the other constituents of the plant present in the extract.

Since capsaicin-induced bronchoconstriction is attributed to release of a mixture of mediators such as the undeca polypeptide-substance P and the neurokinins A and B contained in the pulomonary sensory nerves (Mohammed *et al*., 1993) and to release of histamine (probably from pulmonary mast cells (Krishna and Ghosh, 1990; Pretolani and Vargaftig 1993; Krogstad *et al*., 1999), it is pertinent to suggest that the extract-induced antagonism to capsaicin may be due to its antagonism to the actions of histamine released by capsaicin. The antagonistic action may also involve inhibition of release of substance P and neurokinins A and B or blockade of their receptors. The antagonistic effect to histamine seemed to involve mainly blockade of pulmonary H₁ receptors.

Generally, allergy is considered as a complex and a multifactorial disease that involves formation of many different mediators, the most important of which is histamine. The latter is released from mast cells and basophils resulting in nasal secretion, urticaria, dermatitis, bronchoconstriction and hyotension (White *et al*., 1987, Prefdani and Vargaftig, 1993).

Thus, due to the ability of Fagonia bruguieri freeze-dried aqueous extract to antagonize the bronchoconstriction induced by histamine and capsaicin, we claim the use of this freeze-dried aqueous extract as a component of a pharmaceutical product for the treatment of histamine- and capsaicin-induced bronchoconstriction.

### References

Abdel-Kader, M.S., Omar, A.A., Abdel-Salam, N.A., and Stermitz, F.R. (1993). Erythroxan diterpenes and flavonoids from Fagonia bruguieri. Phytochemistry 33, 718-720.
Abdel-Kader, M.S., Omar, A.A., Abdel-Salam, N.A., Stermitz, F.R. (1994). Erythroxan diterpenes from Fagonia species. Phytochemistry 36, 1431-1433.
Ali, M.A. and Hameed, Z. (1967). Triterpenoids, II. Another sapogenin from Fagonia cretica. Pakistan J. Sci. Industr. Res. 10(2), 140-141.
Al-Wakeel, S.R.M., El-Negoumy, S.I., El-Hadidi, M.N. and Saleh, N.A.M. (1987). Flavonid pattern in Fagonia mollis complex. Biochem. Syst. Ecol. 15, 459-460.
Al-Wakeel S.A.M., El-Cart, I.A. and Saleh, N.A.M. (1988). Distribution of flavonoids in Fagonia thebica complex. Biochem. Syste. Ecol. 16, 57-58.
Al-Wakeel, S.A.M., and Shahnaz, A.M. (1992). Significance of flavonoid chemistry in the Egyptian Fagonia glutinosa and F. isothricha complex. Biochem. Syst. Ecol. 20, 259-64.
Ansari, A.A., Kenne, L., Atta-ur-Rahman, and Wehler, T. (1987). Isolation and characterization of two saponins from Fagonia indica. Phytochem. 26, 1487-90.
Atta-ur-Rahman, Ansari A.A., Drexiex, S.A. and Clardyl, J. (1982). The isolation and structure of nahagenin. Heterocycles 19, 217-20.
Atta-ur-Rahman, Ansari, A.A. and Kenne, L. (1984). Hederagenin, ursolic acid and pinitol from Fagonia indica. J. Natl. Prod. 47, 186-7.
Chopra, R.M., Handa, K.L., Kapur, L.D. and Chopra I.C. (1982). Indigenous Drugs of India. 2nd Edition, Academic Publisher. New Delhi, pp. 507.
El-Hadidi, M.N., Al-Wakeel, S.A.M., E. Cart (1988). Systematic significance of the flavnoid constituents in Fagonia indica complex. Biochem. Syst. Ecol. 16, 293-297.
El-Negoumy, S.I., Al-Wakeel, S.A.M., El Hadidi, M.N. and Saleh N.A.M. (1986). the flavonoids of the Fagonia arabic complex. Phytochem. 25, 2423-4.
ElTahir, K.E.H., Ashoor, M.M. and Al-Harbi, M.M. (1993). The respiratory effects of the black seed (Nigella sativa) in guinea-pigs. Elucidation of the mechanism of action. Gen. Pharmacology 24, 1115-1122.
Ghosh, N. (1984). Fundamentals of Experimental Pharmacology, 2nd Edition. Scientific Book Agency. Calcutta, India.
Hamid, A., Majid, A.C.M. and Atta-ur-Rahman (1989). Isolation of docosyl docosonate from Fagonia cretica Linn. Arab Gulf J. Sci. Res. A. 7, 29-34.
Hash, M.L. and Nag, T. N. (1988). Flavonoids with antimicrobial activities of arid zone plants. Geobios 15, 32-5.
Krishna, De.A., and Ghosh, J.J. (1990). Comparative studies on the involvement of histamine and substance P in the inflammatory response of capsaicin in rat paw. Phytotherapy Res. 4(1), 42-4.
Krogstad, A.L., Lonnroth, P., Larson, G. and Wallin, B.G. (1999). Capsaicin treatment induces histamine release and perfusion changes in psoriatic skin. Br. J. Dermatol. 141, 87-93.
Miyase, J., Melek, F.R., El-Gindi, O.D., Abdel-Khalik, S.M., El-Gindi, M.R., Haggag, M.Y. and Hilal, S.H. (1996). Saponins from Fagonio arabica. Phytochemistry 41(4), 1175-9.
Mohammed, S.P, Higenbottam, T.W., Adcock, J.J. (1993). Effect of aerosol-applied capsaicin, histamine and PGE2 on airway sensory receptors of anaesthetized cats. J. Physiol. (Cambridge), 469, 51-66.
Pretolani, M. and Vargaftig, B.B. (1993). From lung hypersensitivity to bronchial hyperreactivity. What can we learn from studies of animal models? Biochem. Pharmacol. 45, 791-800.
Seed, M.A., Khan, Z. and Sabir, A.W. (1999). Effects of Fagonia cretica L. constituents on various endocrinological parameters in rabbits. Turkish J. Biol. 23(2), 187-197.
Shaker, K.H., Bemhardt, M., Elgamal, M., Hani, A., Seiferet, K. (1999). Triterpenoid saponins from Fagonia indica. Phytochemistry 51(8), 1049-1053.
Toker, J.E., Durham, S.K. Wetton, A.F. and Selig, W.M. (1990). Phospholipase A2-induced pulmonary and hemodynamic responses in guinea-pig. Effects of enzyme inhibitors and mediator antagonists. Am. Rev. Resp. Dis. 142, 1193-1199.
Wagner, H. (1983). Plant Drug Analysis. Springer-Verlag, Berlin Heidelberg.
White, M.V., Slater, J.E. and Kaliner, M.A. (1987). Histamine and Asthma. Am. Rev. Resp. Dis. 135, 1165-1176.

## Claims

1. Freeze-dried aqueous extract of Fagonia bruguieri for use in the treatment of allergic bronchoconstriction.

## Patentansprüche

1. Gefriergetrockneter wässriger Extrakt aus *Fagonia bruguieri* zur Verwendung in der Behandlung von allergischer Bronchienverengung.

## Revendications

1. Extrait aqueux lyophilisé de *Fagonia bruguieri* pour une utilisation dans le traitement de la bronchoconstriction allergique.
